# EUROPÄISCHE PATENTANMELDUNG

(11) **EP 3 563 811 A1**
(43) Veröffentlichungstag der Anmeldung: **06.11.2019**
(21) Anmeldenummer: 19169541.0
(22) Anmeldetag: 16.04.2019
(51) Int. Cl.: A61F 2/915, A61L 29/08, A61L 27/34, A61L 27/52

(54) **VERMINDERUNG DER RISSBILDUNG IN POLYMERSCHICHTEN VON ABBAUBAREN SCAFFOLDS**

(30) Priorität: 03.05.2018 DE 102018110582
(71) Anmelder: BIOTRONIK AG, 8180 Bülach (CH)
(72) Erfinder: Kay, Robert, 18059 Rostock (DE); Forkmann, Christoph, 18057 Rostock (DE); Bayer, Ullrich, 18209 Bad Doberan (DE)
(74) Vertreter: Randoll, Sören

(57) **Zusammenfassung**

Die Erfindung betrifft eine Gefäßstütze (1), wobei die Gefäßstütze (1) einen mit einer Beschichtung (2) beschichteten Grundkörper (10) aufweist, der sich in einer axialen Richtung (x) erstreckt, wobei der Grundkörper (10) eine Mehrzahl an miteinander verbundenen Streben (100) aufweist, wobei die jeweilige Strebe (100) zumindest einen gekrümmten Abschnitt (101) aufweist, der eine konkave Seite (101a) und eine konvexe Seite (101b) aufweist, und wobei die Gefäßstütze (1) von einem Ausgangszustand in einen expandierten Zustand überführbar ist, wobei eine Krümmung des jeweiligen gekrümmten Abschnitts (101) im expandierten Zustand gegenüber dem Ausgangszustand verringert ist. Erfindungsgemäß ist vorgesehen, dass an der jeweiligen konkaven Seite (101a) eine Oberflächenstruktur (3) vorgesehen ist, auf die die Beschichtung (2) aufgebracht ist, und/oder dass zwischen der Beschichtung (2) und dem jeweiligen gekrümmten Abschnitt (101) des Grundkörpers (10) eine Schicht (4) eines Platzhaltermaterials angeordnet ist.

## Beschreibung

Die Erfindung betrifft eine Gefäßstütze, insbesondere eine biodegradierbare Gefäßstütze, die einen Grundkörper aufweist, der aus einer Magnesiumlegierung gebildet ist und mit einer Beschichtung beschichtet ist, die insbesondere ein Polymer aufweist.

Derartige Gefäßstützen werden z.B. verwendet, um ein verengtes Gefäß eines Patienten offenzuhalten bzw. die umgebende Gefäßwand zu stützen und können z.B. mittels eines Ballonkatheters in bekannter Weise implantiert werden. Mögliche Einsatzgebiete können im peripheren, koronaren, kranialen, renalen Bereich und in der Eustachi'ischen Röhre liegen.

Die Beschichtung auf einer derartigen Gefäßstütze, die auch als Scaffold bezeichnet wird, erfüllt neben der Medikamentenfreisetzung noch eine weitere Funktion: Durch Behinderung des Ionenaustauschs zwischen Blut und dem Magnesium des Grundkörpers wird die Degradationsgeschwindigkeit verringert. Häufig kommt es beim Dilatieren von Scaffolds zu Rissen in der Beschichtung, wodurch diese Schutzwirkung beeinträchtigt, die Degradationsgeschwindigkeit zumindest lokal erhöht und damit letztlich die Stützzeit des Scaffolds verringert wird. Die Rissentstehung beruht mit hoher Wahrscheinlichkeit darauf, dass - insbesondere in den Innenseiten bzw. an den konkaven Seiten der gekrümmten bzw. bogenförmigen Abschnitte der Gefäßstütze - beim Dilatieren mechanische Spannungen in der Beschichtung entstehen, die deren plastisches Verformungsvermögen überschreiten. Mögliche lokale Anhaftungen der Beschichtung auf der Scaffoldoberfläche verstärken dieses Problem noch.

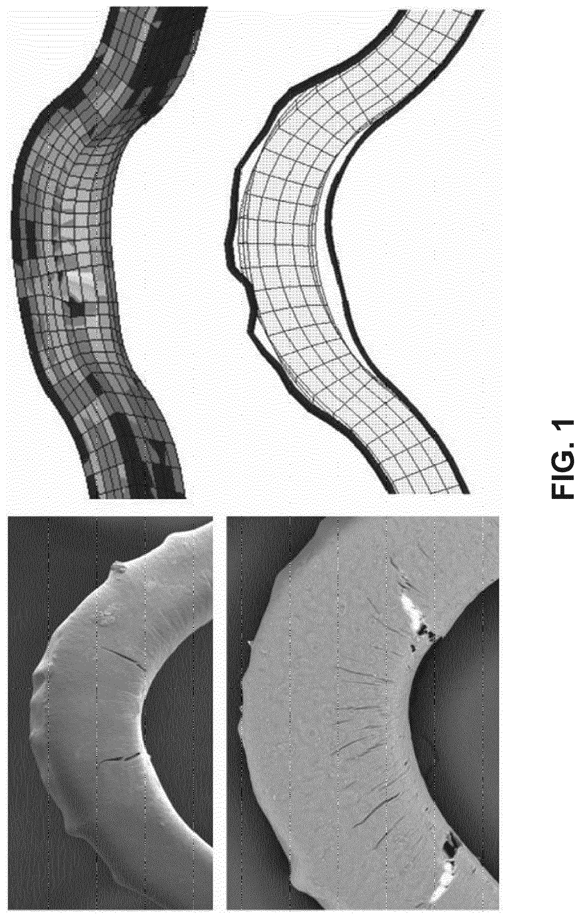

Elektronenmikroskopische Aufnahmen zeigen, dass die Risse hauptsächlich im Bereich der konkaven Schneidflanke der Mäanderbögen auftreten (vgl. Fig. 1 links). FE-Simulationen des Aufweitverhaltens (vgl. Fig. 1 rechts) identifizieren dort kritische Stellen mit hoher Zugbelastung in der Beschichtung bzw. Polymer, was vermutlich zu den erwähnten Rissen führt.

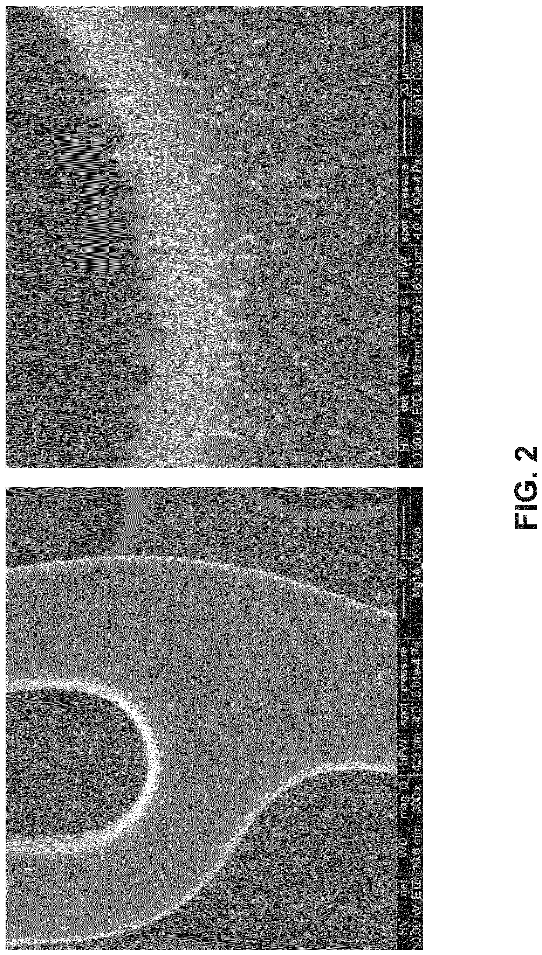

In Fig. 2 ist des Weiteren ein Extrembeispiel für eine raue Scaffoldoberfläche zu sehen, bei der eine ungewollt starke Anhaftung der Polymerschicht zu erwarten ist. Durch die Behinderung von Ausweichbewegungen der Beschichtung bzw. Polymerschicht sind dadurch noch höhere Belastungen derselben zu erwarten.

Bisherige Lösungsansätze zielen z. B. auf eine Begrenzung des erlaubten Dilatationsdurchmessers oder eine Entwicklung belastbarerer Polymere zur Beschichtung des Grundkörpers ab.

Wird die Rissbildung in der Beschichtung bzw. im Polymer lediglich in Kauf genommen, was grundsätzlich erwogen werden könnte, geht dies augenblicklich mit einer unerwünschten verringerten Stützzeit einher, bei der das Potential des Implantats nicht voll ausgeschöpft wird.

Eine Begrenzung des Dilatationsdurchmessers führt zu einer geringeren Ausnutzung der ohnehin schon vergleichsweise niedrigen Materialgrenzen des Magnesium-Werkstoffs des Grundkörpers und erhöht die Gefahr eine Überbeanspruchung des Scaffolds (insbesondere der Beschichtung).

Die Bereitstellung belastbarerer Polymere für die Beschichtung erfordert einen beträchtlichen regulatorischen Aufwand bei der Produktzulassung. Darüber hinaus stellt die mechanische Belastbarkeit nur eine von vielen Anforderungen an ein solches Beschichtungspolymer dar und kann damit nicht beliebig erhöht werden.

Hiervon ausgehend liegt der vorliegenden Erfindung die Aufgabe zugrunde, eine Beschichtung der Gefäßstütze bereitzustellen, derart, dass lokale Spannungsspitzen sowie insbesondere Anhaftungen der Beschichtung vermieden werden und die Beschichtung eine geringere Anfälligkeit für eine Rissbildung aufweist.

Diese Aufgabe wird durch eine Gefäßstütze mit den Merkmalen des Anspruchs 1 gelöst. Vorteilhafte Ausgestaltungen der Erfindung sind in den Unteransprüchen angegeben und werden nachfolgen detailliert beschrieben.

Gemäß Anspruch 1 wird eine Gefäßstütze zum Implantieren in ein Gefäß eines Patienten offenbart, wobei die Gefäßstütze einen mit einer Beschichtung beschichteten Grundkörper aufweist, der sich in einer axialen Richtung erstreckt, wobei der Grundkörper eine Mehrzahl an miteinander verbundenen Streben aufweist, wobei die jeweilige Strebe zumindest einen gekrümmten Abschnitt aufweist, der eine konkave Seite und eine konvexe Seite aufweist, und wobei die Gefäßstütze von einem Ausgangszustand in einen expandierten Zustand überführbar ist, wobei eine Krümmung des jeweiligen gekrümmten Abschnitts im expandierten Zustand gegenüber dem Ausgangszustand verringert ist.

Erfindungsgemäß ist nun vorgesehen, dass zur Verringerung einer Rissbildung der Beschichtung im Bereich des jeweiligen gekrümmten Abschnitts an der jeweiligen konkaven Seite eine Oberflächenstruktur vorgesehen ist, auf die die Beschichtung aufgebracht ist, und/oder dass zwischen der Beschichtung und dem jeweiligen gekrümmten Abschnitt des Grundkörpers eine Schicht aus einem Platzhaltermaterial angeordnet ist.

Bei der Gefäßstütze handelt es sich vorzugsweise um eine biodegradierbare Gefäßstütze. D.h., die implantierte Gefäßstütze ist dazu ausgebildet, sich im Körper des Patienten innerhalb eines insbesondere definierten Zeitraums zu zersetzen. Die Beschichtung kann des Weiteren zumindest einen Stoff aufweisen, der im implantierten Zustand der Gefäßstütze in den Körper des Patienten abgegeben wird und ein Medikament darstellt, zum Beispiel zur Verhinderung einer Restenose (sogenannter Drug Eluting Stent oder DES). Hier sind vornehmlich Sirolimus und dessen manigfaltigen Derivate und Paclitaxel zu nennen. Ferner kann die Beschichtung dazu ausgebildet sein, die Zeitdauer der Biodegradation des Grundkörpers in definierter Weise zu verlängern. In einer bevorzugten Ausführungsform ist die Gefäßstütze ein einstückiger Stent.

Gemäß einer Ausführungsform der Erfindung ist vorgesehen, dass die Gefäßstütze von dem Ausgangszustand in einen expandierten Zustand überführbar ist, indem die Gefäßstütze in radialer Richtung expandiert wird. Die jeweilige radiale Richtung steht dabei senkrecht auf der axialen Richtung.

Gemäß einer Ausführungsform der Erfindung ist vorgesehen, dass das Platzhaltermaterial dazu ausgebildet ist, im implantierten Zustand fließfähig zu sein.

Weiterhin ist gemäß einer Ausführungsform vorgesehen, dass das Platzhaltermaterial bei Körpertemperatur (d.h. 37°C) fließfähig ist, und dass das Platzhaltermaterial bei Raumtemperatur (insbesondere 20°C) fest ist.

Die Oberflächenstruktur der jeweiligen konkaven Seite dient zur Erhöhung der effektiven Oberfläche, so dass entlang einer Erstreckungsrichtung des jeweiligen gekrümmten Abschnitts vergleichsweise mehr Beschichtungsmaterial an der jeweiligen konkaven Seite aufbringbar ist bzw. aufgebracht ist. Beim Expandieren bzw. Dilatieren kann sich die Beschichtung in diesem Bereich ablösen, und statt durch Dehnung der Beschichtung, die zur Rissbildung führen kann, ergibt sich die Streckung in der Erstreckungsrichtung des jeweiligen gekrümmten Abschnitts durch Glattziehen des zusätzlichen bzw. gewellten Beschichtungsmaterials. Alternativ oder ergänzend kann durch das Platzhaltermaterial erreicht werden, dass sich die durch die Beschichtung gebildete Hülle beim Expandieren der Gefäßstütze in radialer Richtung quer zur Erstreckungsrichtung des jeweiligen gekrümmten Abschnitts bewegen kann, wobei das Platzhaltermaterial um den jeweiligen gekrümmten Abschnitt herumfließt, so dass entlang der Erstreckungsrichtung übermäßige hohe Dehnungen der Beschichtung vermieden werden.

Weiterhin ist gemäß einer Ausführungsform der Erfindung vorgesehen, dass die jeweilige Oberflächenstruktur durch jeweils eine Oberfläche gebildet ist, die alternierend angeordnete Erhöhungen und Vertiefungen aufweist.

Bei der Oberflächenstruktur handelt es sich bevorzugt um eine regelmäßige bzw. geordnete Oberflächenstruktur, wobei z.B. eine Breite der jeweiligen Vertiefung und/oder eine Breite der jeweiligen Erhöhung (auf halber Höhe bzw. Tiefe) entlang der besagten Erstreckungsrichtung im Bereich von 1% bis 200%, bevorzugt von 1% bis 90% der Stegbreite, weiter bevorzugt im Bereich von 15 bis 65% der Stegbreite liegt. Weiterhin kann eine Höhe der jeweiligen Erhöhung (relativ zu einem Fußpunkt der Erhöhung) und/oder eine Tiefe der jeweiligen Vertiefung im Bereich von 1% bis 100%, bevorzugt von 1% bis 35% der Stegbreite liegen.

Gemäß einer Ausführungsform der Erfindung ist vorgesehen, dass die jeweilige Oberfläche gewellt ist. Der Vorteil einer solchen Ausführungsform liegt darin, dass die Beschichtung dann auch mit einer Wellenform versehen werden kann, die bei einer Streckung der Beschichtung und einer Ablösung vom Scaffold ohne zu reißen zu einem gewissen Grad gestreckt werden kann, ähnlich einem Federprinzip.

Weiterhin ist gemäß einer Ausführungsform der Erfindung vorgesehen, dass die jeweilige Oberfläche durch eine Oberfläche der konkaven Seite des mindestens einen gekrümmten Abschnitts der jeweiligen Strebe gebildet ist.

Weiterhin ist gemäß einer alternativen Ausführungsform der Erfindung vorgesehen, dass die jeweilige Oberfläche durch eine Oberfläche eines separaten Elements gebildet ist, wobei das jeweilige separate Element an der konkaven Seite des mindestens einen gekrümmten Abschnitts der jeweiligen Strebe festgelegt ist.

Weiterhin ist gemäß einer Ausführungsform der Erfindung vorgesehen, dass die jeweilige Schicht aus dem Platzhaltermaterial im Ausgangszustand der Gefäßstütze überwiegend oder vollständig auf der konkaven Seite des mindestens einen gekrümmten Abschnitts der jeweiligen Strebe angeordnet ist.

Alternativ hierzu kann vorgesehen sein, dass die jeweilige Schicht des Platzhaltermaterials im Ausgangszustand der Gefäßstütze den gekrümmten Abschnitt der jeweiligen Strebe im Querschnitt umgibt.

Die Schichten des Platzhaltermaterials an den konkaven Seiten bzw. um den jeweiligen gekrümmten Abschnitt der Streben herum, können auch zusammenhängend ausgebildet sein, d.h., miteinander verbunden sein.

Gemäß einer Ausführungsform ist der gesamte Grundkörper mit einer zusammenhängenden Schicht aus dem Platzhaltermaterial umgeben. D.h., die Schichten bilden eine zusammenhängende, den Grundkörper umgebende Schicht. Auf diese Schicht ist dann insbesondere die besagte Beschichtung aufgebracht.

Weiterhin ist gemäß einer Ausführungsform der Erfindung vorgesehen, dass die jeweilige Schicht des Platzhaltermaterials dazu ausgebildet ist, im implantierten Zustand der Gefäßstütze sowie beim Überführen der Gefäßstütze in den expandierten Zustand zumindest teilweise von der konvexen Seite auf die konkave Seite des mindestens einen gekrümmten Abschnitts der jeweiligen Strebe zu fließen bzw. dorthin verdräng zu werden. Hierdurch können übermäßige Spannungen/Dehnungen der Beschichtung im Bereich der gekrümmten Abschnitte der Streben verhindert werden, was das Risiko einer Rissbildung deutlich vermindert.

Weiterhin ist gemäß einer Ausführungsform der Erfindung vorgesehen, dass die jeweilige Strebe eine Mehrzahl an miteinander verbundenen gekrümmten Abschnitten aufweist, so dass die jeweilige Strebe einen mäanderförmigen Verlauf aufweist, wobei der jeweilige gekrümmten Abschnitt eine konkave Seite und eine konvexe Seite aufweist, und wobei die Gefäßstütze in radialer Richtung expandierbar ausgebildet ist, so dass eine Krümmung des jeweiligen gekrümmten Abschnitts verringert wird, wenn die Gefäßstütze in radialer Richtung expandiert wird, und wobei die Gefäßstütze von einem Ausgangszustand in einen expandierten Zustand überführbar ist, wobei eine Krümmung des jeweiligen gekrümmten Abschnitts im expandierten Zustand gegenüber dem Ausgangszustand verringert ist, und wobei zur Verringerung einer Rissbildung der Beschichtung im Bereich des jeweiligen gekrümmten Abschnitts an der jeweiligen konkaven Seite eine Oberflächenstruktur vorgesehen ist, auf die die Beschichtung aufgebracht ist, und/oder dass zwischen der Beschichtung und dem jeweiligen gekrümmten Abschnitt des Grundkörpers eine Schicht aus einem Platzhaltermaterial angeordnet ist.

Die jeweilige Oberflächenstruktur kann wiederum gemäß einer der oben beschriebenen Ausführungsformen ausgebildet sein. Gleiches gilt hinsichtlich der jeweiligen Schicht des Platzhaltermaterials. D.h., an der jeweiligen konkaven Seite bzw. an dem jeweiligen gekrümmten Abschnitt kann Platzhaltermaterial in der oben bereits beschriebenen Weise vorgesehen sein.

Weiterhin ist gemäß einer Ausführungsform der vorliegenden Erfindung vorgesehen, dass die jeweilige Strebe in einer Umfangsrichtung des Grundkörpers umläuft.
Weiterhin kann gemäß einer Ausführungsform der vorliegenden Erfindung vorgesehen sein, dass die jeweilige Strebe mit einer in axialer Richtung benachbarten Strebe über zumindest einen entlang der axialen Richtung erstreckten Steg verbunden ist. Vorzugsweise ist die jeweilige umlaufende sowie insbesondere mäanderförmige Strebe über je zwei Stege mit einer benachbarten Strebe verbunden.

Weiterhin ist gemäß einer Ausführungsform der Erfindung vorgesehen, dass das Platzhaltermaterial durch einen der folgenden Stoffe gebildet ist oder einen der folgenden Stoffe aufweist: ein Hydrogel, ein thermoreversibles Hydrogel, ein thermoreversibles Hydrogel, das bei einer Körpertemperatur flüssig ist und das bei Raumtemperatur fest ist. Ein Beispiel für ein Hydrogel is Polyacrylamide. Geeignet als Platzhaltermaterial ist ferner eine Mischung aus Polyethylenglykol/Poly-L-lactid.

Weiterhin ist gemäß einer Ausführungsform der Erfindung vorgesehen, dass das Platzhaltermaterial durch eine Trennschicht von der Beschichtung getrennt ist.

Gemäß einer Ausführungsform der Erfindung kann die Trennschicht einen der folgenden Stoffe aufweisen oder aus einem der folgenden Stoffe gebildet sein: Magnesiumstearat, Zinkstearat, Lithiumstearat.

Weiterhin ist gemäß einer Ausführungsform der Erfindung vorgesehen, dass die Beschichtung durch einen der folgenden Stoffe gebildet ist oder einen der folgenden Stoffe aufweist: ein Polymer wie Polylactid, insbesondere Poly-L-lactid, Polylactid-co-Glycolid, Polycaprolacton oder Kombinationen daraus (Blends, Co-Polymere). Es ist somit ersichtlich, dass es bevorzugt ist, wenn die Beschichtung wie der Scaffold biodegradierbar ist.

Weiterhin ist gemäß einer Ausführungsform der Erfindung vorgesehen, dass der Grundkörper aus einem der folgenden Stoffe gebildet ist oder einen der folgenden Stoffe aufweist: eine Magnesiumlegierung, z.B. legiert mit Seltenen Erden (z.B. WE43) oder Magnesium-Aluminium-Legierungen.

Die Magnesiumlegierung ist insbesondere gemäß einer Ausführungsform so gewählt, dass der Grundkörper (insbesondere über einen definierten Zeitraum hinweg) biodegradierbar bzw. im Körper des Patienten zersetzbar ist.

Ein weiterer Aspekt der vorliegenden Erfindung betrifft ein Verfahren zur Herstellung einer erfindungsgemäßen Gefäßstütze, wobei der Grundkörper der Gefäßstütze bereitgestellt wird und wobei der jeweilige gekrümmte Abschnitt auf der konkaven Seite die jeweilige Oberflächenstruktur erhält, und/oder wobei auf den jeweiligen gekrümmten Abschnitt des Grundkörpers eine Schicht eines Platzhaltermaterials aufgebracht wird, und wobei anschließend die Beschichtung aufgebracht wird.

Die Oberflächenstrukturen bzw. die besagten Schichten können bei dem Verfahren in den oben bereits beschriebenen Weisen angeordnet bzw. ausgebildet werden.

Weitere Merkmale und Ausführungsformen der vorliegenden Erfindung sollen nachfolgend anhand der Figuren erläutert werden. Es zeigen:
- Fig. 1: elektronenmikroskopische Aufnahmen (links) und FE-Simulationen (rechts) der Beschichtung auf dilatierten Magnesium-Gefäßstützen;
- Fig. 2: elektronenmikroskopische Aufnahmen von elektropolierten Magnesium-Gefäßstützen mit rauer Oberfläche. Die Rauheit wird durch intermetallische Verbindungen der Legierungselemente mit Magnesium hervorgerufen. Diese treten beim Elektropolieren an die Oberfläche und bewirken somit eine unerwünscht hohe Adhäsion zur nachfolgend aufzubringenden Beschichtung (Polymerschicht);
- Fig. 3: durch die Verformung des Grundkörpers der Gefäßstütze in der Beschichtung erzeugte Zugbelastungen: Zug entlang der konkaven Seite bzw. Flanke (A), Zug über die Strebenbreite (B);
- Fig. 4: eine schematische Darstellung einer Ausführungsform einer erfindungsgemäßen Gefäßstütze mit wellenförmiger Oberfläche bzw. Kontur an der konkaven Seite des jeweiligen gekrümmten Abschnitts bzw. Mäanderbogens des Grundkörpers zur Erzeugung einer Beschichtung mit Wellen als Dehnungsreserve;
- Fig. 5: eine schematische Darstellung eines gekrümmten Abschnitts eines Grundkörpers einer Ausführungsform einer erfindungsgemäßen Gefäßstütze, wobei das Platzhaltermaterial initial auf der konvexen Seite des gekrümmten Abschnitts angeordnet ist;
- Fig. 6: eine schematische Darstellung eines gekrümmten Abschnitts eines Grundkörpers einer weiteren Ausführungsform einer erfindungsgemäßen Gefäßstütze, wobei das Platzhaltermaterial initial auf alle Seiten des gekrümmten Abschnitts verteilt ist; und
- Fig. 7: eine perspektivische Ansicht einer Ausführungsform einer erfindungsgemäßen Gefäßstütze.

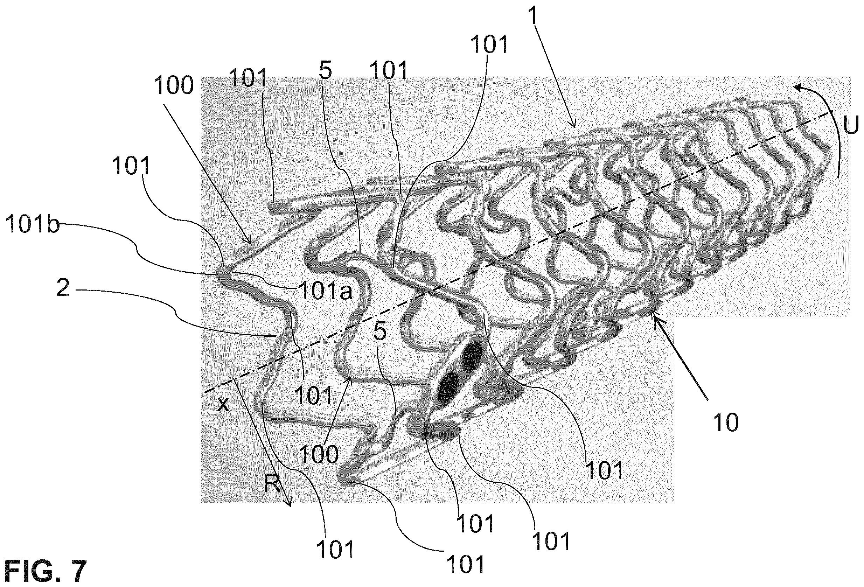

Figur 7 zeigt eine Ausführungsform eine erfindungsgemäßen Gefäßstütze 1, wobei die Gefäßstütze 1 einen mit einer Beschichtung 2 beschichteten Grundkörper 10 aufweist, der sich in einer axialen Richtung x erstreckt, wobei der Grundkörper 10 eine Mehrzahl an miteinander verbundenen Streben 100 aufweist, wobei die jeweilige Strebe 100 zumindest einen gekrümmten Abschnitt 101, bevorzugt mehrere derartige Abschnitte 101, aufweist, wobei der jeweilige gekrümmte Abschnitt 101 eine konkave Seite 101a und eine konvexe Seite 101b aufweist, und wobei die Gefäßstütze 1 von einem Ausgangszustand in einen expandierten Zustand überführbar ist, wobei eine Krümmung des jeweiligen gekrümmten Abschnitts 101 im expandierten Zustand gegenüber dem Ausgangszustand verringert ist. Erfindungsgemäß ist nun vorgesehen, dass zur Verringerung einer Rissbildung der Beschichtung 2 im Bereich des jeweiligen gekrümmten Abschnitts 101 an der jeweiligen konkaven Seite 101a eine Oberflächenstruktur 3 vorgesehen ist, auf die die Beschichtung 2 aufgebracht ist, und/oder dass zwischen der Beschichtung 2 und dem jeweiligen gekrümmten Abschnitt 101 des Grundkörpers 10 eine Schicht 4 eines Platzhaltermaterials angeordnet ist.

Die jeweilige Strebe 100 des Grundkörpers 10 kann in einer Umfangsrichtung U des Grundkörpers 10 umlaufen, wobei die gekrümmten Abschnitte 101 so ausgebildet sind, dass sie - bezogen auf die axiale Richtung x - alternierend angeordnete Minima und Maxima bilden, also eine mäanderförmige Struktur bilden. Weiterhin kann die jeweilige Strebe 100 mit einer in der axialen Richtung x benachbarten Strebe 100 über z.B. zwei Stege 5 verbunden sein.

Das erfindungsgemäße Vorsehen von Oberflächenstrukturen 3 und/oder Platzhaltermaterialschichten 4 wird insbesondere aufgrund der folgenden Problematik vorgenommen.

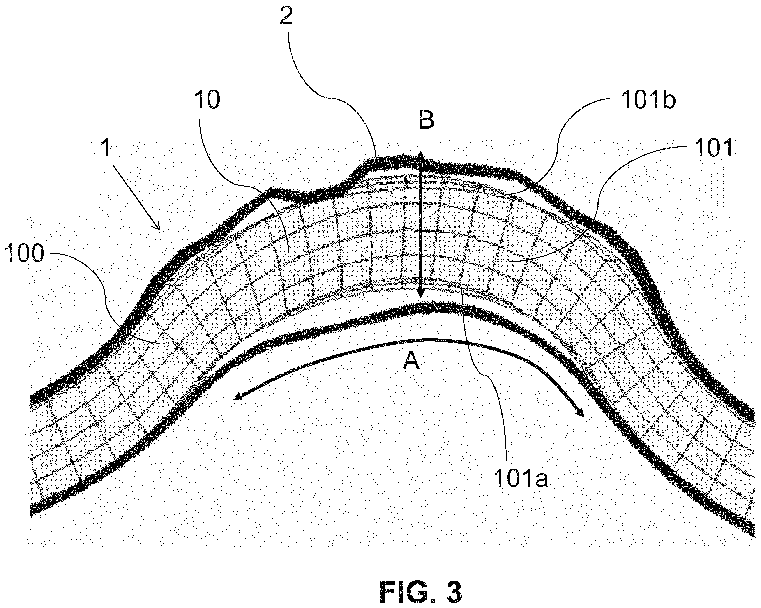

Das Aufbiegen der gekrümmten Abschnitte 101, die insbesondere als Mäanderbögen 101 ausgestaltet sind, beim Dilatieren bzw. Expandieren der Gefäßstütze 1 in radialer Richtung R, sorgt auf zwei Arten für Zugbelastung in der Beschichtung 2, die in der Fig. 3 dargestellt sind, nämlich einerseits durch Dehnung des Beschichtungsmaterials 2 auf der jeweiligen konkaven Seite bzw. Flanke 101a der gekrümmten Abschnitt 101, d.h., auf der Zugseite der Biegestruktur in Richtung A, sowie andererseits durch die Bestrebung der Beschichtung 2, sich von der jeweiligen konkaven Seite 101a abzulösen (B), was dadurch behindert wird, dass die Beschichtung 2 jeweils die gesamte Strebe 100 bzw. den Grundkörper 10 umschließt. Die beiden Mechanismen sind insbesondere gekoppelt: wenn mehr Ablösung von der jeweiligen konkaven Seite 101a möglich ist (ohne zu hohe Zugbelastung im Bereich B), dann wird die Zugbelastung entlang der Richtung A geringer sein.

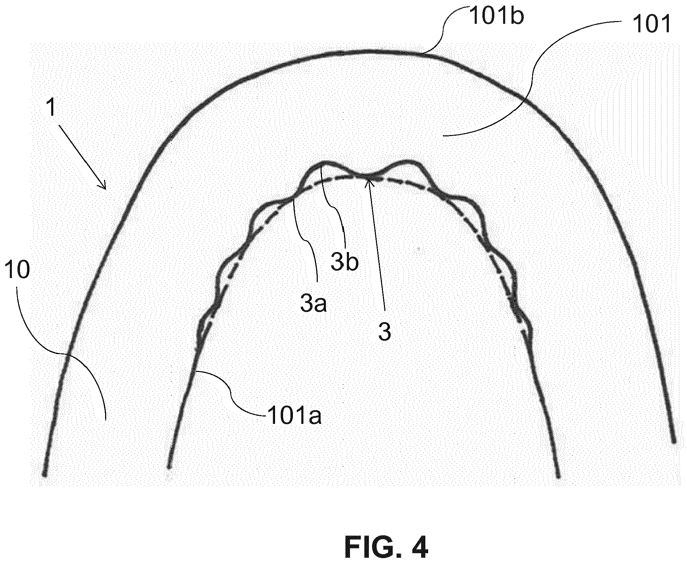

Eine Möglichkeit, entlang der Richtung A für mehr unverformte Materiallänge der Beschichtung 2 zu sorgen, ist in Fig. 4 dargestellt. Hier ist die jeweilige konkave Seite 101a mit einer gewellten Oberfläche 3 ausgestattet. Beim Aufbringen der Beschichtung 2 legt sich die Beschichtung 2 auf der jeweiligen konkaven Seite 101a an die gewellte Kontur bzw. Oberfläche 3 an, was im Vergleich zum üblichen Konturverlauf (Fig. 4: gestrichelt) zu mehr Material entlang der Richtung A (vgl. Fig. 3) führt. Beim Expandieren in radialer Richtung R löst sich die Beschichtung 2 insbesondere in diesem Bereich ab, und statt durch Dehnung des Beschichtungsmaterials 2 ergibt sich die Streckung in Richtung A durch Glattziehen der Wellen. Anstatt direkt eine gewellte Geometrie des Scaffoldgrundkörpers 10 zu verwenden, kann die gewellte Kontur bzw. Oberfläche 3 auch jeweils mit einem separaten Element, z.B. mit einem wachsartigen Material, erzeugt werden.

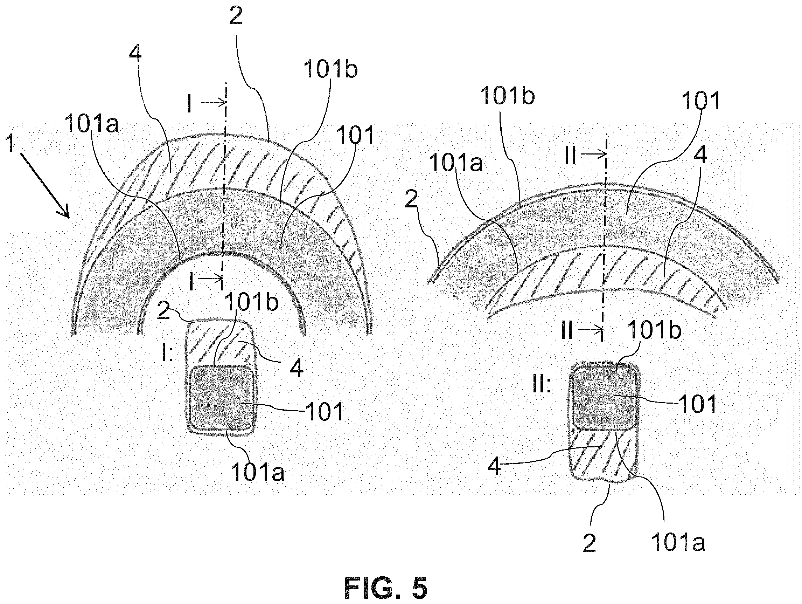

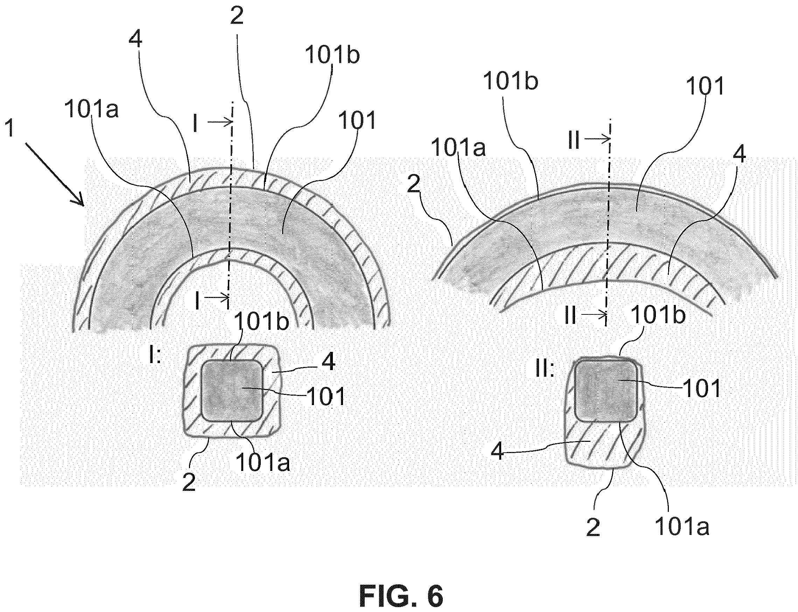

Eine weitere Ausführungsform der Erfindung, die in den Figuren 5 bzw. 6 dargestellt ist, setzt an der Richtung B an (vgl. Fig. 3). Vor der Beschichtung 2 wird auf den Grundkörper 10 ein Platzhaltermaterial 4 aufgebracht, welches an den gewünschten Stellen für einen Abstand zwischen Grundkörpermaterial 10 und Beschichtung 2 sorgt, und welches insbesondere Eigenschaften (z.B. weich, vorzugsweise viskos) besitzt, die ein Fließen des Platzhaltermaterials zwischen der Beschichtung 2 und dem Grundkörper 10 um den jeweiligen gekrümmten Abschnitt 101 herum erlaubt.

Aus mechanischer Sicht ist die in Fig. 5 gezeigte Ausführung besonders vorteilhaft. Dabei wird das Platzhaltermaterial gezielt nur auf den konvexen Seiten 101a der gekrümmten Abschnitte bzw. Mäanderbögen 101 in Form je einer Schicht 4 aufgebracht (vgl. Fig. 5, linke Seite). Während der Dilatation fließt das Platzhaltermaterial 4 um den jeweiligen gekrümmten Abschnitt 101 der betreffenden Strebe 100 herum (vgl. Fig. 5, rechte Seite), so dass sich die gesamte Beschichtung 2 in Richtung B (vgl. Fig. 3) bewegen kann, ohne dass in Richtung A (vgl. Fig. 3) übermäßig hohe Dehnungen in der Beschichtung auftreten. Weiterhin zeigt Fig. 6 eine alternative Ausführungsform, die gegenüber der in der Figur 5 gezeigten Ausführungsform den Vorteil einer vereinfachten Herstellung der Gefäßstütze 1 aufweist, da hier eine gezielte Aufbringung des Platzhaltermaterials 4 auf die konkaven Seiten 101a umgangen wird, indem das Platzhaltermaterial bzw. eine daraus gebildete Schicht 4 auf allen Seiten des jeweiligen gekrümmten Abschnitts 101 des Grundkörpers 10 vorgesehen wird, insbesondere auf der konkaven und auf der konvexen Seite 101a, 101b, so dass der jeweilige Abschnitt101 bzw. die jeweilige Strebe 100 im Querschnitt vom Platzhaltermaterial bzw. einer entsprechenden Schicht 4 umgeben ist (vgl. Fig. 6, linke Seite). Diese Schicht 4 kann z.B. mittels eines Tauch- oder Sprühverfahrens auf den Grundkörper 10 aufgebracht werden. Auch in diesem Fall sorgt das Fließen des Platzhaltermaterials um die jeweilige Strebe 100 bzw. den jeweiligen Abschnitt 101 herum (Fig. 6, rechte Seite) dafür, dass sich die Beschichtung 2 in Richtung B (vgl. Fig. 3) bewegen kann, wenn die Gefäßstütze in der radialen Richtung expandiert wird, ohne dass in der Richtung A (vgl. Fig. 3) übermäßig hohe Dehnungen in der Beschichtung 2 auftreten.

Als Platzhaltermaterial wird bevorzugt ein thermoreversibles Hydrogel verwendet, z.B. Polyacrylamid oder Polyethylenglykol/Poly-L-lactid. Solche Hydrogele zeichnen sich dadurch aus, dass sie in gewissen Temperaturbereichen gelieren, d.h. eine gewisse Festigkeit aufweisen, in anderen Temperaturbereichen dagegen wieder flüssig werden. Bevorzugt ist das erfindungsgemäße Platzhaltermaterial bzw. Hydrogel so zusammengesetzt, dass es bei Raumtemperatur fest und bei Körpertemperatur flüssig ist. Eine Anpassung der entsprechenden Übergangstemperaturen kann z.B. durch Auswahl eines oder mehrerer Hydrogele aus der Vielzahl der möglichen Gele sowie durch Einstellung des Mischungsverhältnisses der Gele erfolgen.

Ein sich bei Körpertemperatur verflüssigendes Platzhaltermaterial wirkt als Trennmittel zwischen der Beschichtung 2 und dem Grundkörper 10 auch unerwünschten lokalen Anhaftungen entgegen. Als Trennschicht zwischen dem Platzhaltermaterial und der Beschichtung 2 (insbesondere Polymer), z.B. um eine Vermischung zu vermeiden, kann z.B. ein Magnesiumstearat verwendet werden.

Die vorliegende Erfindung ermöglicht insbesondere durch eine Verringerung bzw. Vermeidung der Rissbildung in der Beschichtung 2 des Grundkörpers 10 der Gefäßstütze 1, der insbesondere aus einer Magnesiumlegierung besteht, die Stützzeit der Gefäßstütze 1 zu verlängern. Mit der erfindungsgemäßen Lösung ist dies insbesondere unter Verwendung der bekannten und regulatorisch unkritischen Polymermaterialien für die Beschichtung 2 möglich.

## Patentansprüche

1. Gefäßstütze (1), wobei die Gefäßstütze (1) einen mit einer Beschichtung (2) beschichteten Grundkörper (10) aufweist, der sich in einer axialen Richtung (x) erstreckt, wobei der Grundkörper (10) eine Mehrzahl an miteinander verbundenen Streben (100) aufweist, wobei die jeweilige Strebe (100) zumindest einen gekrümmten Abschnitt (101) aufweist, der eine konkave Seite (101a) und eine konvexe Seite (101b) aufweist, und wobei die Gefäßstütze (1) von einem Ausgangszustand in einen expandierten Zustand überführbar ist, wobei eine Krümmung des jeweiligen gekrümmten Abschnitts (101) im expandierten Zustand gegenüber dem Ausgangszustand verringert ist,
**dadurch gekennzeichnet,**
**dass** an der jeweiligen konkaven Seite (101a) eine Oberflächenstruktur (3) vorgesehen ist, auf die die Beschichtung (2) aufgebracht ist, und/oder dass zwischen der Beschichtung (2) und dem jeweiligen gekrümmten Abschnitt (101) des Grundkörpers (10) eine Schicht (4) eines Platzhaltermaterials angeordnet ist.

2. Gefäßstütze nach Anspruch 1, **dadurch gekennzeichnet, dass** die jeweilige Oberflächenstruktur (3) durch eine Oberfläche (3) gebildet ist, die alternierend angeordnete Erhöhungen (3a) und Vertiefungen (3b) aufweist.

3. Gefäßstütze nach Anspruch 2, **dadurch gekennzeichnet, dass** die jeweilige Oberfläche (3) gewellt ist.

4. Gefäßstütze nach Anspruch 2 oder 3, **dadurch gekennzeichnet, dass** die jeweilige Oberfläche (3) durch eine Oberfläche der konkaven Seite (101a) des mindestens einen gekrümmten Abschnitts (101) der jeweiligen Strebe (100) gebildet ist.

5. Gefäßstütze nach Anspruch 2 oder 3, **dadurch gekennzeichnet, dass** die jeweilige Oberfläche durch eine Oberfläche eines separaten Elements gebildet ist, wobei das jeweilige separate Element an der konkaven Seite (101a) des mindestens einen gekrümmten Abschnitts (101) der jeweiligen Strebe (100) festgelegt ist.

6. Gefäßstütze nach einem der vorhergehenden Ansprüche, **dadurch gekennzeichnet, dass** die jeweilige Schicht (4) des Platzhaltermaterials im Ausgangszustand der Gefäßstütze (1) überwiegend oder vollständig auf der konvexen Seite (101b) des mindestens einen gekrümmten Abschnitts (101) der jeweiligen Strebe (100) angeordnet ist.

7. Gefäßstütze nach einem Ansprüche 1 bis 5, **dadurch gekennzeichnet, dass** die jeweilige Schicht (4) des Platzhaltermaterials im Ausgangszustand der Gefäßstütze (1) den mindestens einen gekrümmten Abschnitt (101) der jeweiligen Strebe (100) umgibt.

8. Gefäßstütze nach Anspruch 6 oder 7, **dadurch gekennzeichnet, dass** das die jeweilige Schicht (4) des Platzhaltermaterials dazu ausgebildet ist, im implantierten Zustand der Gefäßstütze (1) sowie beim Überführen der Gefäßstütze (1) in den expandierten Zustand zumindest teilweise von der konvexen Seite (101b) auf die konkave Seite (101a) des mindestens einen gekrümmten Abschnitts (101) der jeweiligen Strebe (100) zu fließen.

9. Gefäßstütze nach einem der vorhergehenden Ansprüche, **dadurch gekennzeichnet, dass** die jeweilige Strebe (100) eine Mehrzahl an miteinander verbundenen gekrümmten Abschnitten (101) aufweist, so dass die jeweilige Strebe (100) einen mäanderförmigen Verlauf aufweist, wobei der jeweilige gekrümmten Abschnitt (101) eine konkave Seite (101a) und eine konvexe Seite (101b) aufweist, und wobei die Gefäßstütze (1) von einem Ausgangszustand in einen expandierten Zustand überführbar ist, wobei eine Krümmung des jeweiligen gekrümmten Abschnitts (101) im expandierten Zustand gegenüber dem Ausgangszustand verringert ist, und wobei an der jeweiligen konkaven Seite (101a) eine Oberflächenstruktur (3) vorgesehen ist, auf die die Beschichtung (2) aufgebracht ist, und/oder dass zwischen der Beschichtung (2) und dem jeweiligen gekrümmten Abschnitt (101) des Grundkörpers (10) eine Schicht (4) aus einem Platzhaltermaterial angeordnet ist.

10. Gefäßstütze nach einem der vorhergehenden Ansprüche, **dadurch gekennzeichnet, dass** die jeweilige Strebe (100) in einer Umfangsrichtung (U) des Grundkörpers (10) umläuft.

11. Gefäßstütze nach einem der vorhergehenden Ansprüche, **dadurch gekennzeichnet, dass** die jeweilige Strebe (100) mit einer benachbarten Strebe (100) über zumindest einen entlang der axialen Richtung (x) erstreckten Steg (5) verbunden ist.

12. Gefäßstütze nach einem der vorhergehenden Ansprüche, **dadurch gekennzeichnet, dass** das Platzhaltermaterial durch einen der folgenden Stoffe gebildet ist oder einen der folgenden Stoffe aufweist: ein Hydrogel, ein thermoreversibles Hydrogel, ein thermoreversibles Hydrogel, das bei einer Körpertemperatur flüssig ist und das bei Raumtemperatur fest ist, bevorzugt Polyacrylamid oder Polyethylenglykol/Poly-L-lactid.

13. Gefäßstütze nach einem der vorhergehenden Ansprüche, **dadurch gekennzeichnet, dass** das Platzhaltermaterial durch eine Trennschicht von der Beschichtung (2) getrennt ist.

14. Gefäßstütze nach einem der vorhergehenden Ansprüche, **dadurch gekennzeichnet, dass** die Beschichtung (2) durch einen der folgenden Stoffe gebildet ist oder einen der folgenden Stoffe aufweist: ein Polymer wie Polylactid, insbesondere Poly-L-lactid, Polylactid-co-Glycolid, Polycaprolacton oder Kombinationen daraus.

15. Gefäßstütze nach einem der vorhergehenden Ansprüche, **dadurch gekennzeichnet, dass** der Grundkörper (10) aus einem der folgenden Stoffe gebildet ist oder einen der folgenden Stoffe aufweist: Magnesium oder eine Magnesiumlegierung.
